(19) Europäisches Patentamt
European Patent Office
Office européen des brevets

(11) Veröffentlichungsnummer : **0 582 021 B1**

# (12) EUROPÄISCHE PATENTSCHRIFT

(45) Veröffentlichungstag der Patentschrift :
**13.07.94 Patentblatt 94/28**

(51) Int. Cl.[5] : **C07D 401/12**, A01N 47/36

(21) Anmeldenummer : **92810582.4**

(22) Anmeldetag : **30.07.92**

(54) **Sulfonylharnstoffe als Herbizide.**

(43) Veröffentlichungstag der Anmeldung :
**09.02.94 Patentblatt 94/06**

(45) Bekanntmachung des Hinweises auf die Patenterteilung :
**13.07.94 Patentblatt 94/28**

(84) Benannte Vertragsstaaten :
**AT BE CH DE DK ES FR GB GR IT LI LU MC NL PT SE**

(56) Entgegenhaltungen :
**EP-A- 0 103 543**

(73) Patentinhaber : **CIBA-GEIGY AG**
**Klybeckstrasse 141**
**CH-4002 Basel (CH)**

(72) Erfinder : **Schurter, Rolf, Dr.**
**Holzmattstrasse 45**
**CH-4102 Binningen (CH)**
Erfinder : **Föry, Werner, Dr.**
**Inzlingerstrasse 11**
**CH-4125 Riehen (CH)**

**EP 0 582 021 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, herbizid wirksame N-Pyridylsulfonyl-N'-pyrimidinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Phenylsulfonylharnstoffe mit herbizider Wirkung sind aus der Europäischen Patentanmeldung Nr. 0 103 543 bekannt. Die dort konkret offenbarten Wirkstoffe können jedoch die Anforderungen bezüglich Wirkungsstärke und Wirkungsspektrum nicht immer erfüllen. Es besteht somit ein Bedarf nach besser wirksamen und selektiveren Wirkstoffen.

Es wurden nun neue Sulfonylharnstoffe mit verbesserten herbiziden Eigenschaften gefunden.

Die neuen Sulfonylharnstoffe ensprechen der Formel I

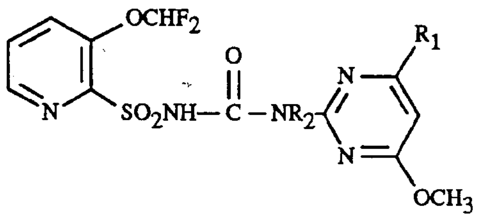

(I)

worin

$R_1$ für Methyl oder Methoxy und

$R_2$ für Wasserstoff oder Methyl stehen; sowie die Salze dieser Verbindungen mit Aminen, Alkali- oder Erdalkalimetallbasen oder mit quaternären Ammoniumbasen.

Die Verbindungen der Formel I können Salze bilden, bei denen der Wasserstoff der $-SO_2$-NH-Gruppe durch ein für die Landwirtschaft geeignetes Kation ersetzt wird. Diese Salze sind beispielsweise Metallsalze, insbesondere Alkali- oder Erdalkalisalze oder auch Ammoniumsalze oder Salze mit organischen Aminen.

Als Beispiele für zur Ammonium-Kationenbildung geeignete Amine kommen sowohl Ammoniak wie auch primäre, sekundäre und tertiäre $C_1$-$C_{18}$-Alkylamine, $C_1$-$C_4$-Hydroxyalkylamine und $C_2$-$C_4$-Alkoxyalkylamine in Betracht, beispielsweise Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-iso-propylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Trimethylamin, Triethylamin, Tri-n-propylamin, Tri-iso-propylamin, Tri-n-butylamin, Tri-iso-butylamin, Tri-sek.-butylamin, Tri-n-amylamin, Methoxyethylamin und Ethoxyethylamin; heterocyclische Amine wie z.B. Pyridin, Chinolin, iso-Chinolin, Morpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Triethylamin, iso-Propylamin und Di-iso-propylamin.

In den Verbindungen der Formel I steht $R_2$ vorzugsweise für Wasserstoff. Als bevorzugte Einzelverbindung aus dem Umfang der Formel I ist zu nennen:

N-(3-Difluormethoxypyridin-2-yl-sulfonyl)-N'-(4-methyl-6-methoxypyrimidin-2-yl)harnstoff.

Die Verbindungen der Formel I können hergestellt werden, indem man

a) ein Pyridylsulfonamid der Formel II

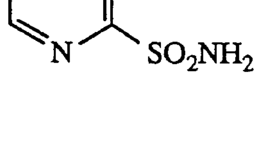

(II)

mit einem N-Pyrimidinylcarbamat der Formel III

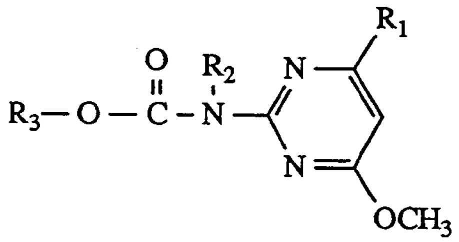

(III)

worin $R_1$ und $R_2$ die unter Formel I angegebene Bedeutung haben und $R_3$ Phenyl oder 4-Tolyl bedeutet, in Gegenwart einer Base umsetzt, oder

b) eine Verbindung der Formel IV

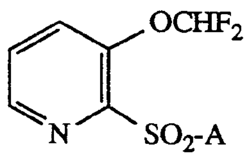

(IV)

worin A

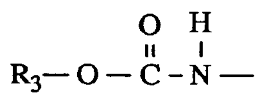

oder O=C=N- bedeutet, wobei $R_3$ die oben angegebene Bedeutung hat, in Gegenwart einer Base mit einem 2-Aminopyrimidin der Formel V

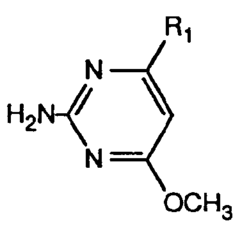

(V)

worin $R_1$ die unter Formel I angegebene Bedeutung hat, umsetzt, oder

c) ein Pyridylsulfonamid der Formel II

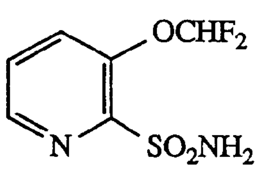

(II)

in Gegenwart einer Base mit einem Pyrimidinylisocyanat der Formel VI

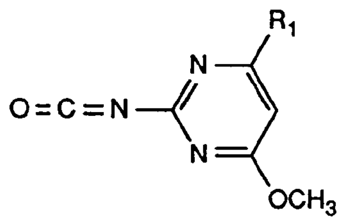

(VI)

worin $R_1$ die unter Formel I angegebene Bedeutung hat, umsetzt, oder
d) eine Verbindung der Formel VII

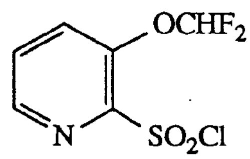

(VII)

mit einer Verbindung der Formel V

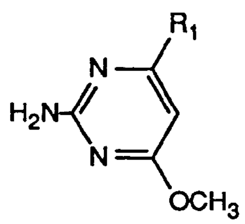

(V)

worin $R_1$ die unter Formel I angegebene Bedeutung hat, in Gegenwart eines Ammonium-, Phosphonium-, Sulfonium- oder Alkalimetallcyanatsalzes der Formel VIII

$M^{+}OCN^{-}$ (VIII)

worin M für ein Alkalimetall oder für die Gruppe $R_4$ $R_5$ $R_6$ $R_7$Q steht, worin $R_4$, $R_6$, $R_6$ und $R_9$ unabhängig voneinander $C_1$-$C_{18}$-Alkyl, Benzyl oder Phenyl bedeutet, wobei die Gesamtzahl der C-Atome nicht größer als 36 ist; und Q Stickstoff, Schwefel oder Phosphor bedeutet, umsetzt.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die Verbindungen der Formeln II, III, IV, V, VI, VII und VIII sind entweder bekannt oder lassen sich analog bekannter Verfahren herstellen. Die Verfahrensvariante a) ist beispielsweise in EP-A0 103 543, die Verfahrensvariante b) beispielsweise in EP-A-0 101 670 beschrieben. Die Verfahrensvarianten c) und d) sind in EP-A-0 459 949 bzw. dem Schweizer Patent Nr. 662 348 offenbart.

Die Wirkstoffe der Formel I werden in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen, wobei der Einsatz in Maiskultur ganz besonders bevorzugt ist.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemäßen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Straßenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodaß die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindung der Formel I oder sie enthaltender Mittel zur Regulierung des Pflanzenwachstums kommen verschiedene Methoden und Techniken in Betracht, wie beispielsweise die folgenden:

i) <u>Samenbeizung</u>

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.

b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).

c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 0,001 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) <u>Kontrollierte Wirkstoffabgabe</u>

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, die Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und

Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

Emulgierbare Konzentrate:

| | |
|---|---|
| Aktiver Wirkstoff: | 1 bis 90 %, vorzugsweise 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Herstellung der Verbindungen der Formel I wird durch die folgenden Beispiele näher erläutert.

Beispiel H1: Herstellung von 2-Isopropylthio-3-hydroxypyridin:

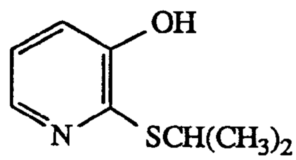

Zu einer Lösung von 16,4 g 2-Mercapto-3-hydroxypyridin (bekannt aus Tetrahedron **21**, 2191, (1980)) in 155 ml Dimethylformamid gibt man bei einer Temperatur von +5°C portionsweise 14 g Kalium-tert.-butylat hinzu. Anschließend gibt man bei einer Temperatur von 0 bis +2°C 12,5 ml Isopropyljodid innerhalb von 15 Minuten tropfenweise hinzu. Nach 2-stündigem Rühren bei Raumtemperatur wird das Reaktionsgemisch mit 400 ml Eiswasser und 200 ml Ethylacetat extrahiert. Nach Einstellen des pH-Wertes auf 7 bis 8 mit 2N Salzsäure wäscht man die wäßrige Phase viermal mit je 150 ml Ethylacetat. Die vereinigten organischen Phasen werden dreimal mit je 100 ml Wasser gewaschen, am Vakuum auf ein Volumen von 100 ml eingeengt und viermal mit je 50 ml 2N wäßrigem Natriumhydroxidlösung bei einer Temperatur von 0°C extrahiert. Anschließend wird die wäßrige Phase mit 2N Salzsäure auf einen pH-Wert von 7 bis 8 eingestellt und viermal mit je 50 ml Ethylacetat extrahiert. Nach Trocknen der organischen Phase, Filtration über 100 g Kieselgel, viermaligem Waschen mit je 50 ml Ethylacetat und eindampfen erhält man einen kristallisierenden Rückstand, der mit Petrolether verrieben und anschließend filtriert wird. Man erhält 14,6 g 2-Isopropylthio-3-hydroxypyridin mit einem Schmelzpunkt von 64°C.

Beispiel H2: Herstellung von 2-Isopropylthio-3-difluormethoxypyridin:

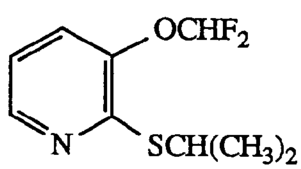

Zu einer Lösung von 118,4 g 2-Isopropylthio-3-hydroxypyridin in 560 ml Dioxan gibt man innerhalb von 15 Minuten 464 ml 30%-ige wäßrige Natriumhydroxidlösung tropfenweise hinzu. Anschließend leitet man bei einer Temperatur von 80°C innerhalb von 2 Stunden 121 g Freon-22 ein und rührt für weitere 90 Minuten. Nach Abkühlen der Reaktionsmischung auf Raumtemperatur extrahiert man mit einer Mischung aus 2500 ml Eiswasser und 1000 ml Methylenchlorid und wäscht die Phasen viermal mit je 50 ml Methylenchlorid und einmal mit 100 ml Eiswasser. Anschließend werden die vereinigten organischen Phasen über Natriumsulfat getrocknet und im Vakuum eingedampft. Nach Reinigung über Kieselgelchromatographie mit Ethylacetat/n-Hexan 1 : 9 als Eluenten erhält man 105,7 g 2-Isopropylthio-3-difluormethoxypyridin in Form eines Öls mit einem $n_D^{21}$ von 1,5088.

Beispiel H3: Herstellung von 3-Difluormethoxypyridin-2-yl-sulfonamid:

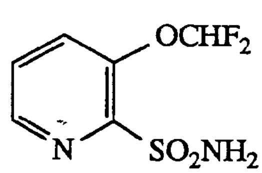

Zu einer Mischung von 105,7 g 2-Isopropylthio-3-difluormethoxypyridin, 1000 ml Dichlormethan und 1723 ml 1N Salzsäure leitet man innerhalb von 50 Minuten bei einer Temperatur von -5 bis 0°C 142 g gasförmiges Chlor ein. Nach 20-minütigem Rühren bei einer Temperatur von -30°C leitet man innerhalb von 15 Minuten Stickstoff ein. Anschließend werden die Phasen dreimal mit je 250 ml Eiswasser und 250 ml Dichlormethan gewaschen und die vereinigten organischen Phasen über Natriumsulfat getrocknet. Diese Reaktionsmischung wird nun bei einer Temperatur von -50 bis -40°C innerhalb von 40 Minuten zu einer Mischung von 122,7 g Ammoniak in 250 ml Dichlormethan tropfenweise zugegeben. Nach 15-stündigem Rühren und anschließendem

Filtrieren wird die Reaktionsmischung eigedampft, mit Petrolether verrieben, das erhaltene Kristallagglomerat filtriert und getrocknet. Man erhält 85,9 g 3-Difluormethoxypyridin-2-yl-sulfonamid mit einem Schmelzpunkt von 85 bis 86°C.

Beispiel H4: Herstellung von N-(3-Difluormethoxypyridin-2-yl-sulfonyl)-N'-(4-methyl-6-methoxypyrimidin-2-yl)-harnstoff (Verbindung Nr. 1.03):

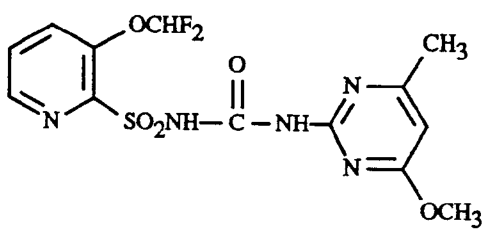
(1.03)

Zu einer Lösung von 4,03 g 3-Difluormethoxypyridin-2-yl-sulfonamid in 40 ml Acetonitril gibt man nacheinander 4,97 g N-(4-Methyl-6-methoxypyrimidin-2-yl)-phenylcarbamat und 2,95 ml 1,5-Diazabicyclo[5.4.0]undec-5-en hinzu. Nach 60-minütigem Rühren wird die Reaktionsmischung im Vakuum eingeengt. Anschließend wird der so erhaltene ölige Rückstand mit 12 ml 2N Salzsäure verrieben und mit 10 ml Wasser verdünnt. Das kristallisierte Produkt wird filtriert und anschließend nacheinander mit Wasser und Diethylether gewaschen. Man erhält N-(3-Difluormethoxypyridin-2-yl-sulfonyl)-N'-(4-methyl-6-methoxypyrimidin-2-yl)-harnstoff (Verbindung 1.03) mit einem Schmelzpunkt von 151 bis 154 °C.

In analoger Weise werden die in der angeschlossenen Tabelle 1 aufgelisteten Verbindungen der Formel I sowie deren Zwischenprodukte hergestellt.

Tabelle 1: Verbindungen der Formel I:

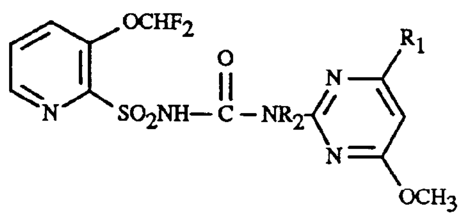
(I)

| Verb. Nr. | $R_1$ | $R_2$ | Phys. Daten |
|---|---|---|---|
| 1.01 | $OCH_3$ | H | Smp.137-139°C |
| 1.02 | $OCH_3$ | $CH_3$ | |
| 1.03 | $CH_3$ | H | Smp.151-154°C |
| 1.04 | $CH_3$ | $CH_3$ | |

Biologische Beispiele

Beispiel B1: Herbizidwirkung vor dem Auflaufen der Pflanzen

Kunststofftöpfe werden mit expandiertem Vermiculit (Dichte: 0,135 g/cm³, Wasseradsorptionsvermögen: 0,565 l/l) gefüllt. Nach dem Sättigen des nicht adsorptiven Vermiculits mit einer wäßrigen Wirkstoffemulsion in deionisiertem Wasser, die die Wirkstoffe in einer Konzentration von 70 ppm enthält, werden Samen der folgenden Pflanzen auf die Oberfläche gesät: Nasturtium officinalis, Agrostis tenuis, Stellaria media und Digitaria sanguinalis. Die Versuchsgefäße werden anschließend in einer Klimakammer bei einer Temperatur von 20 °C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Gießwasser 0,5 % eines handelsüblichen Flüssigdüngers zugesetzt. 12 Tage nach der Aussaat wird der Versuch ausgewertet und die Wirkung auf die Versuchspflanzen nach dem folgenden Maßstab bewertet:

| | |
|---|---|
| 1 | :Pflanze nicht gekeimt oder total abgestorben |
| 2-3 | :sehr starke Wirkung |
| 4-6 | :mittlere Wirkung |
| 7-8 | :schwache Wirkung |
| 9 | :keine Wirkung (wie unbehandelte Kontrolle) |

Tabelle B1: preemergente Wirkung:

Konzentration der Wirkstoffemulsion: 70 ppm

| Testpflanze: Wirkstoff Nr. | Nasturtium | Stellaria | Agrostis | Digitaria |
|---|---|---|---|---|
| 1.03 | 4 | 3 | 2 | 2 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wäßrigen Wirkstoffdispersion in einer Dosierung von 8-500 g Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet. Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Formel I starke Herbizidwirkung.

Formulierungsbeispiele für Wirkstoffe der Formel I (% = Gewichtsprozent)

| 1. Spritzpulver | a) | b) | c) |
|---|---|---|---|
| Wirkstoff gemäß Tabelle 1 | 20 % | 50 % | 0,5 % |
| Na-Ligninsulfonat | 5 % | 5 % | 5 |
| Na-Laurylsulfat | 3 % | - | - % |
| Na-Diisobutylnaphthalinsulfonat | - | 6 % | 6 % |
| Octylphenolpolyethylenglykolether (7-8 Mol AeO) | - | 2 % | 2 |
| Hochdisperse Kieselsäure | 5 % | 27 % | 27 % |
| Kaolin | 67 % | - % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrate | a) | b) |
|---|---|---|
| Wirkstoff gemäß Tabelle 1 | 10% | 1 % |
| Ca-Dodecylbenzolsulfonat | 3 % | 3 % |
| Octylphenolpolyethylenglykolether (4-5 Mol AeO) | 3 % | 3 % |
| Ricinusöl-polyethylenglykolether (36 Mol AeO) | 4 % | 4 |
| Cyclohexanon | 30 | 10 % |
| Xylolgemisch | 50 % | 79 % |

Aus solchen Konzentraten können durch Verdünnen mit Wasser Emulsionen jeder gewünschten Konzentration hergestellt werden.

| 3. Stäubemittel | a) | b) |
|---|---|---|
| Wirkstoff gemäß Tabelle 1 | 0,1 % | 1 % |
| Talkum | 99,9 % | - |
| Kaolin | - | 99 % |

Durch inniges Vermischen der Trägerstoffe mit dem Wirkstoff erhält man gebrauchsfertige Stäubemittel.

| 4. Extruder-Granulat | a) | b) |
|---|---|---|
| Wirkstoff gemäß Tabelle 1 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschließend im Luftstrom getrocknet.

5. Umhüllungs-Granulat

| | |
|---|---|
| Wirkstoff gemäß Tabelle 1 | 3 % |
| Polyäthylenglykol (MG200) | 3% |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 6. Suspensions-Konzentrat | a) | b) |
|---|---|---|
| Wirkstoff gemäß Tabelle 1 | 5 % | 40 % |
| Ethylenglykol | 10 % | 10 % |
| Nonylphenolpolyethylenglykolether (15 Mol AeO) | 1 % | 6 % |
| Na-Ligninsulfonat | 5 % | 10 % |
| Carboxymethylcellulose | 1 % | 1 % |
| 37 %ige wäßrige Formaldehyd-Lösung | 0,2 % | 0,2 % |
| Silikonöl in Form einer 75%-igen wäßrigen Emulsion | 0,8 % | 0,8 % |
| Wasser | 77 % | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 7. Salzlösung | |
|---|---|
| Wirkstoff gemäß Tabelle 1 | 5 % |
| Isopropylamin | 1 % |
| Octylphenolpolyethylenglycol-ether (78 Mol AeO) | 91 % |

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

**Patentansprüche**

1. N-Pyridylsulfonyl-N'-pyrimidinylharnstoffe der Formel I

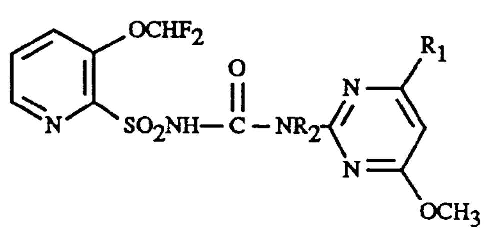

(I)

worin
$R_1$ für Methyl oder Methoxy und
$R_2$ für Wasserstoff oder Methyl stehen sowie und die Salze dieser Verbindungen mit Aminen, Alkali- oder Erdalkalimetallbasen oder mit quaternären Ammoniumbasen.

**2.** Verbindungen nach Anspruch 1, dadurch gekennzeichnet, daß $R_2$ für Wasserstoff steht.

**3.** N-(3-Difluormethoxypyridin-2-yl-sulfonyl)-N'-(4-methyl-6-methoxypyrimidin-2-yl)-harnstoff nach Anspruch 1.

**4.** Verfahren zur Herstellung der Verbindungen der Formel I gemäß Anspruch 1, dadurch gekennzeichnet, daß man

a) ein Pyridylsulfonamid der Formel II

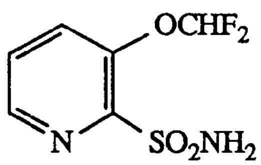

(II)

mit einem N-Pyrimidinylcarbamat der Formel III

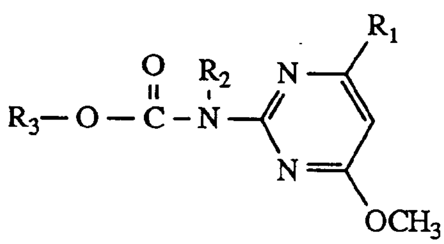

(III)

worin $R_1$ und $R_2$ die unter Formel I angegebene Bedeutung haben und $R_3$ Phenyl oder 4-Tolyl bedeutet, in Gegenwart einer Base umsetzt, oder

b) eine Verbindung der Formel IV

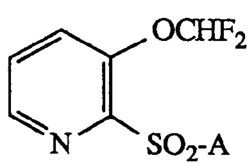

(IV)

worin A

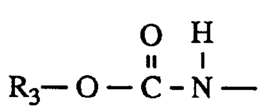

oder O=C=N- bedeutet, wobei $R_3$ die oben angegebene Bedeutung hat, in Gegenwart einer Base mit einem 2-Aminopyrimidin der Formel V

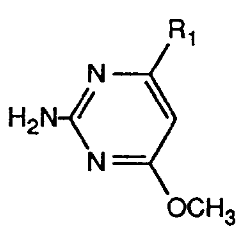

(V)

worin $R_1$ die unter Formel I angegebene Bedeutung hat, umsetzt, oder
c) ein Pyridylsulfonamid der Formel II

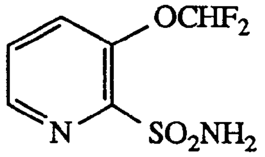

(II)

in Gegenwart einer Base mit einem Pyrimidinylisocyanat der Formel VI

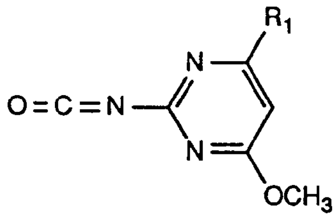

(VI)

worin $R_1$ die unter Formel I angegebene Bedeutung hat, umsetzt, oder
d) eine Verbindung der Formel VII

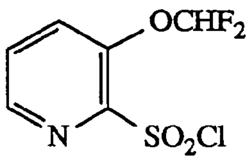

(VII)

mit einer Verbindung der Formel V

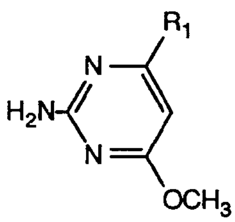

(V)

worin $R_1$ die unter Formel I angegebene Bedeutung hat, in Gegenwart eines Ammonium-, Phosphonium-, Sulfonium- oder Alkalimetallcyanatsalzes der Formel VIII

$M^+OCN^-$ (VIII)

worin M für ein Alkalimetall oder für die Gruppe $R_4$ $R_5$ $R_6$ $R_7$Q steht, worin $R_4$, $R_5$, $R_6$ und $R_9$ unabhängig voneinander $C_1$-$C_{18}$-Alkyl, Benzyl oder Phenyl bedeutet, wobei die Gesamtzahl der C-Atome nicht größer als 36 ist; und Q Stickstoff, Schwefel oder Phosphor bedeutet, umsetzt.

**5.** Ein herbizides und den Pflanzenwuchs hemmendes Mittel, gekennzeichnet durch ein Gehalt an einem oder mehreren Sulfonylharnstoffen der Formel I, gemäß Anspruch 1.

**6.** Mittel gemäß Anspruch 5, dadurch gekennzeichnet, daß es zwischen 0,1 % und 95 % Wirkstoff der Formel I gemäß Anspruch 1 enthält.

**7.** Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

8. Verfahren gemäß Anspruch 7, dadurch gekennzeichnet, daß man eine Wirkstoffmenge zwischen 0,001 und 2 kg pro Hektar appliziert.

9. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

10. Verfahren gemäß Anspruch 8, zur selektiven pre- oder post-emergenten Bekämpfung von Unkräutern in Nutzpflanzenkulturen.

## Claims

1. An N-pyridylsulfonyl-N′-pyrimidinylurea of formula I

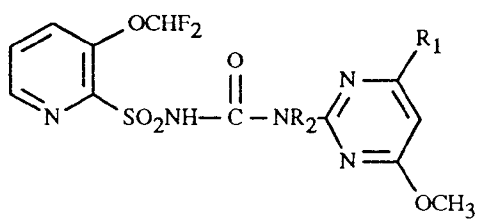

(I)

wherein
$R_1$ is methyl or methoxy and
$R_2$ is hydrogen or methyl,
or a salt of such a compound with an amine, an alkali metal or alkaline earth metal base or with a quaternary ammonium base.

2. A compound according to claim 1 wherein $R_2$ is hydrogen.

3. N-(3-Difluoromethoxypyridin-2-yl-sulfonyl)-N′-(4-methyl-6-methoxypyrimidin-2-yl)urea according to claim 1.

4. A process for the preparation of a compound of formula I according to claim 1, which comprises
a) reacting a pyridylsulfonamide of formula II

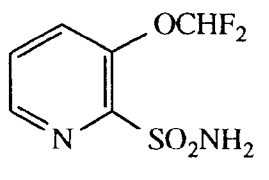

(II)

with an N-pyrimidinylcarbamate of formula III

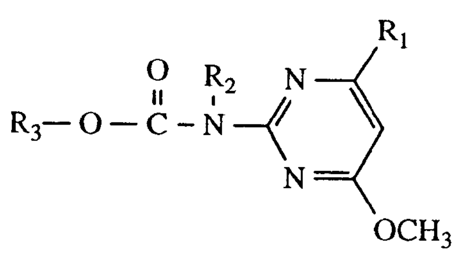

(III)

wherein $R_1$ and $R_2$ are as defined for formula I and $R_3$ is phenyl or 4-tolyl, in the presence of a base,

or

b) reacting a compound of formula IV

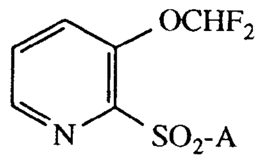

(IV)

wherein A is

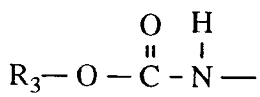

or O=C=N-, wherein $R_3$ is as defined above, in the presence of a base, with a 2-aminopyrimidine of formula V

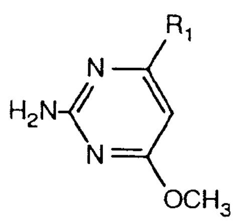

(V)

wherein $R_1$ is as defined for formula I, or

c) reacting a pyridylsulfonamide of formula II

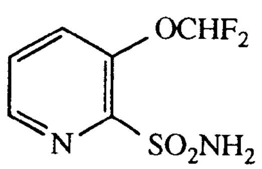

(II),

in the presence of a base, with a pyrimidinyl isocyanate of formula VI

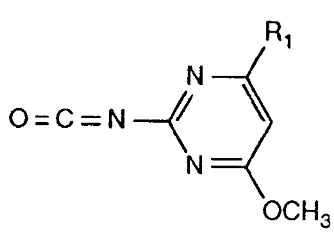

(VI)

wherein $R_1$ is as defined for formula I, or

d) reacting a compound of formula VII

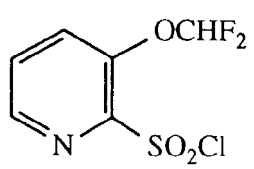

(VII)

with a compound of formula V

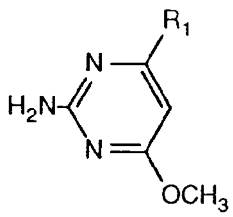

(V)

wherein $R_1$ is as defined for formula I, in the presence of an ammonium, phosphonium, sulfonium or alkali metal cyanate salt of formula VIII

$M^+OCN^-$ (VIII)

wherein M is an alkali metal or the group $R_4$ $R_5$ $R_5$ $R_7$Q, wherein $R_4$, $R_5$, $R_5$ and $R_9$ independently of one another are $C_1$-$C_{18}$alkyl, benzyl or phenyl, the total number of carbon atoms not exceeding 36; and Q is nitrogen, sulfur or phosphorus.

**5.** A herbicidal and plant-growth-inhibiting composition comprising one or more sulfonylureas of formula I, according to claim 1.

**6.** A composition according to claim 5 comprising from 0.1 % to 95 % of a compound of formula I according to claim 1.

**7.** A method of controlling undesirable plant growth, which comprises applying an effective amount of a compound of formula I, according to claim 1, or of a composition comprising that compound, to the plants or to the locus thereof.

**8.** A method according to claim 7, which comprises applying active ingredient in an amount of from 0.001 to 2 kg per hectare.

**9.** A method of inhibiting plant growth, which comprises applying an effective amount of a compound of formula I, according to claim 1, or of a composition comprising that compound, to the plants or to the locus thereof.

**10.** A method according to claim 8 for the selective pre- or post-emergence control of weeds in crops of useful plants.

## Revendications

**1.** N-pyridylsulfonyl-N'-pyrimidinylurées de formule I

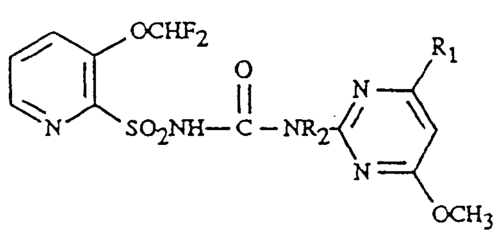

(I)

dans laquelle
$R_1$ représente un groupe méthyle ou méthoxy, et
$R_2$ représente l'hydrogène ou un groupe méthyle,
et les sels de ces composés et d'amines, de bases alcalines ou alcalinoterreuses ou de bases d'ammonium quaternaires.

2. Composés selon la revendication 1, caractérisés en ce que $R_2$ représente l'hydrogène.

3. La N-(3-difluorométhoxypyridine-2-yl-sulfonyl)-N'-(4-méthyl-6-méthoxypyrimidine-2-yl)-urée selon revendication 1.

4. Procédé de préparation des composés de formule I de la revendication 1, caractérisé en ce que :
a) on fait réagir un pyridylsulfonamide de formule II

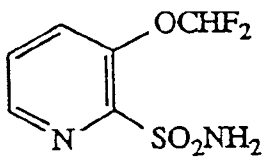

(II)

avec un N-pyrimidinylcarbamate de formule III

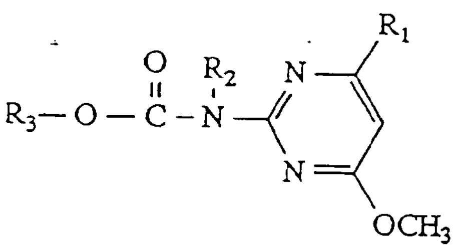

(III)

dans laquelle $R_1$ et $R_2$ ont les significations indiquées en référence à la formule I et $R_3$ représente un groupe phényle ou 4-tolyle, en présence d'une base, ou bien
b) on fait réagir un composé de formule IV

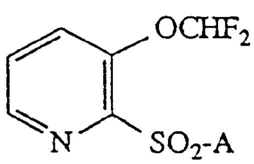

(IV)

dans laquelle A représente

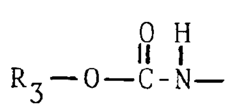

ou O=C=N-, $R_3$ ayant les significations indiquées ci-dessus, en présence d'une base, avec une 2-aminopyrimidine de formule V

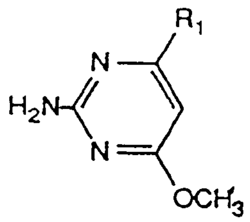

(V)

dans laquelle $R_1$ a les significations indiquées en référence à la formule I, ou bien c) on fait réagir un pyridylsulfonamide de formule II

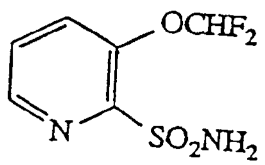

(II)

en présence d'une base, avec un pyrimidinylisocyanate de formule VI

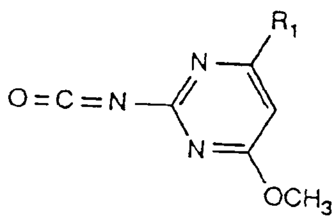

(VI)

dans laquelle $R_1$ a les significations indiquées en référence à la formule I, ou bien d) on fait réagir un composé de formule VII

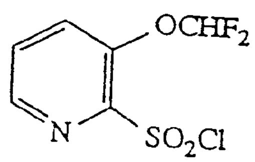

(VII)

avec un composé de formule V

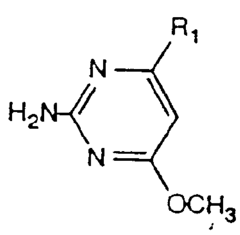

(V)

dans laquelle $R_1$ a les significations indiquées en référence à la formule I, en présence d'un cyanate d'ammonium, de phosphonium, de sulfonium ou de métal alcalin de formule VIII

$M^+OCN^-$ (VIII)

dans laquelle M représente un métal alcalin ou le groupe $R_4R_5R_6R_7Q$ dans lequel $R_4$, $R_5$, $R_6$ et $R_7$ représentent chacun, indépendamment les uns des autres, un groupe alkyle en C 1-C 18, benzyle, ou phényle, le nombre total des atomes de carbone ne dépassant pas 36 ; et Q représente l'azote, le soufre ou le phosphore.

**5.** Un produit herbicide et inhibiteur de la croissance des végétaux, caractérisé en ce qu'il contient une ou plusieurs sulfonylurées de formule I de la revendication 1.

**6.** Produit selon revendication 5, caractérisé en ce qu'il contient de 0,1% à 95% d'une substance active de formule I de la revendication 1.

**7.** Procédé pour combattre les croissances de végétaux indésirables, caractérisé en ce que l'on applique sur les végétaux ou leur habitat, en quantité efficace, une substance active de formule I de la revendication 1 ou un produit contenant une telle substance active.

**8.** Procédé selon revendication 7, caractérisé en ce que l'on applique une quantité de substance active de 0,001 à 2 kg/ha.

**9.** Procédé pour inhiber la croissance des végétaux, caractérisé en ce que l'on applique sur les végétaux ou leur habitat, en quantité efficace, une substance active de formule I de la revendication 1 ou un produit contenant une telle substance active.

**10.** Procédé selon la revendication 8, pour la lutte sélective en pré-levée ou en post-levée contre les végétaux adventices dans les cultures de végétaux utiles.